# EUROPEAN PATENT APPLICATION

(11) **EP 1 065 280 A2**
(43) Date of publication of application: **03.01.2001**
(21) Application number: 00114247.0
(22) Date of filing: 03.07.2000
(51) Int. Cl.: C12Q 1/68

(54) **Dna chip and determination of its sensitivity**

(30) Priority: 02.07.1999 JP 18936099
(71) Applicant: Fuji Photo Film Co., Ltd., Kanagawa 250-0123 (JP)
(72) Inventor: Kuhara, Satoru, Fukuoka-shi, Fukuoka 811-1355 (JP); Tashiro, Kosuke, Fukuoka-shi, Fukuoka 813-0043 (JP); Muta, Shigeru, Fukuoka-shi, Fukuoka 812-0063 (JP); Hakamata, Masashi, c/o Fuji Photo Film Co.,Ltd., Ashigara-kami-gun, Kanagawa 258-8538 (JP); Hora, Naofumi, c/o Fuji Photo Film Co., Ltd., Tokyo 106-8620 (JP)
(74) Representative: Weisert, Annekäte, Dipl.-Ing. Dr.-Ing.

(57) **Abstract**

A DNA chip composed of a substrate which has plural sections on its surface and a group of DNA fragments fixed at their one ends onto each of the sections, a group of DNA fragments fixed onto one section having the same base sequence, wherein a DNA fragment is composed of at least two portions, one portion having a base sequence specifically selected for each section and the other portion having a common base sequence is favorably utilized for accurate base sequencing of a sample DNA fragment because a relative sensitivity on each section can be determined by bringing the DNA chip into contact with a labelled oligonucleotide having a portion of a base sequence which is complementary to the common base sequence of the portion of the DNA fragment, so as to combine the labelled oligonucleotide to each of the DNA fragments through hybridization, and detecting the labelled oligonucleotide combined to the DNA fragment on each section of the DNA chip.

## Description

### FIELD OF THE INVENTION

This invention relates to a DNA chip which is, in particular, favorably employable for analysis of expression, variation and polymorphism of gene.

### BACKGROUND OF THE INVENTION

The gene expression in cells and organs have been analyzed by Northern blotting method (Dot blotting) which comprises the steps of preparing RNA from the cell and organ, fixing the RNA onto a membrane, and performing hybridization using a probe which is specific to the gene to be analyzed, or RT-PCR method utilizing a primer which is specific to the gene to be analyzed.

In the development of gene technology, specifically in the progress of Genome Project, it is recently required to analyze a great number of genes of living organs within a short period of time.

In answer to the above-described requirements, a micro-array method utilizing a DNA chip has been developed. The DNA chip generally comprises a small substrate (e.g., a substrate having 1 cm² surface area) which has plural sections (e.g., sections of several hundreds or several thousands) on its surface and a group of DNA fragments (namely, probe DNA fragments) fixed at their one ends onto each of the sections, a group of DNA fragments fixed onto one section having essentially the same base sequence.

The DNA chip is brought into contact with a small amount of a labelled target DNA fragment so as to perform hybridization between the probe DNA fragments and the target DNA fragment. The DNA chip is utilized for detecting the nature of the target DNA fragments.

The DNA chip is prepared either by an on-chip method (in which the DNA fragments are synthesized from their units on the substrate) or by a method by spotting a solution of a group of previously prepared DNA fragments on the substrate and fixing the fragments onto the substrate by electrostatic bonding or covalent bonding.

In any cases, it is extremely difficult to fix the DNA fragments in the same amounts in all the predetermined sections of the substrate, even though the spottings are made using a solution of the same amounts. For instance, since the amount of the solution of DNA fragments to be spotted is extremely small, it is difficult to adjust the spotting amount accurately in the predetermined level. Moreover, the different DNA fragments sometimes show different fixing ability to the substrate. It also happens that once fixed DNA fragments separate during or after the fixing procedure. The difference of amount of DNA fragments fixed onto different sections brings about difference of detection sensitivity in the DNA fragment-fixed Sections of the DNA chip.

In the conventional use of DNA chip, the sensitivity difference does not bring about serious problems in the analysis of DNA fragments, because the conventional DNA fragment analysis generally does not require quantitative detection. In the recent use of DNA chip, particularly, for the analysis of a DNA fragment of mis-match structure, or for the analysis of a mixture of DNA fragments, it is sometimes required to determine a relative detection sensitivity in each section of the DNA chip.

Until now, there is not known a method for accurately determine the relative detection sensitivity of each section of the very DNA chip to be utilized in the analysis.

### SUMMARY OF THE INVENTION

It is an object of the present invention to provide a DNA chip which is easily determined in the detection sensitivity of each DNA fragment-fixed section of the chip.

It is another object of the invention to provide a method of determining the detection sensitivity of each DNA fragment-fixed section of the DNA chip.

The present invention resides in a DNA chip comprising a substrate which has plural sections on its surface and a group of DNA fragments fixed at their one ends onto each of the sections, a group of DNA fragments fixed onto one section having essentially the same base sequence, wherein a DNA fragment comprises at least two portions, one portion having a base sequence specifically selected for each section and the other portion having a common base sequence.

In the DNA chip of the invention, the DNA fragment preferably comprises three portions, one portion having a base sequence specifically selected for each section, one of the other two portions having a common base sequence and being placed between the substrate and one end of the portion of specifically selected base sequence, and another of the other two porions having a common base sequence and being attached to the other end of the portion of specifically selected base sequence.

The invention also resides in a kit which has a DNA chip comprising a substrate which has plural sections on its surface and a group of DNA fragments fixed at their one ends onto each of the sections, a group of DNA fragments fixed onto one section having essentially the same base sequence, wherein a DNA fragment comprises at least two portions, one portion having a base sequence specifically selected for each section and the other portion having a common base sequence, and a labelled oligonucleotide having a portion of a base sequence which is complementary to the common base sequence of the portion of the DNA fragment fixed onto the substrate.

In the kit of the invention, the labelled oligonucleotide preferably comprises an oligonucleotide and a fluorescence label.

The invention further resides in a method of determining a relative sensitivity on each section, which comprises the steps of:
bringing a DNA chip comprising a substrate which has plural sections on its surface and a group of DNA fragments fixed at their one ends onto each of the sections, a group of DNA fragments fixed onto one section having essentially the same base sequence, wherein a DNA fragment comprises at least two portions, one portion having a base sequence specifically selected for each section and the other portion having a common base sequence, into contact with a labelled oligonucleotide having a portion of a base sequence which is complementary to the common base sequence of the portion of the DNA fragment fixed onto the substrate, in the presence of an aqueous medium, so as to combine the labelled oligonucleotide to each of the DNA fragments through hybridization; and
detecting the labelled oligonucleotide combined to the DNA fragment on each section of the DNA chip.

In the method of the invention, the labelled oligonucleotide preferably comprises an oligonucleotide and a fluorescence label, and the detection is performed by fluorometry.

### BRIEF DESCRIPTION OF DRAWINGS

Fig. 1 schematically shows a DNA chip according to the invention.
Fig. 2 schematically shows one method of determining the relative detection sensitivity of each section according to the invention.
Fig. 3 schematically shows another method of determining the relative detection sensitivity of each section according to the invention.

### DETAILED DESCRIPTION OF THE INVENTION

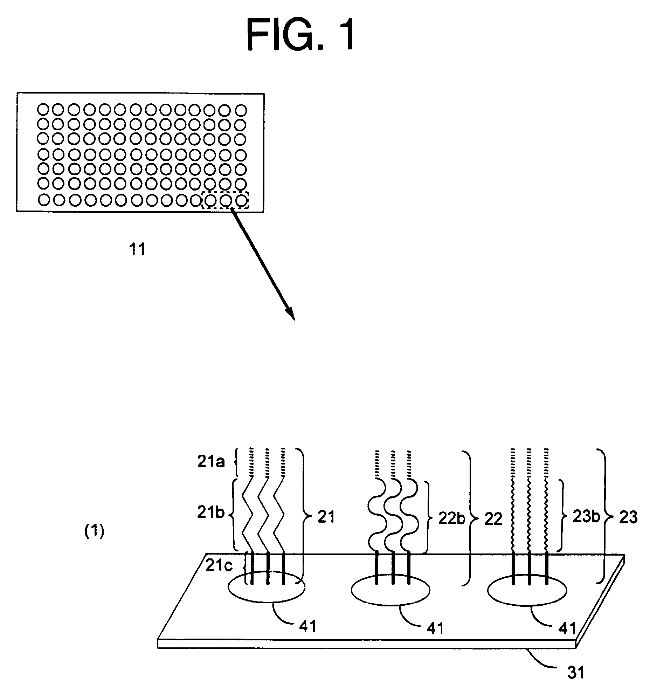

A typical DNA chip of the invention is schematically illustrated in Fig. 1. In Fig. 1, a DNA chip (11) has a substrate (31) on which a plurality of sections (41) for each group of specifically selected DNA fragments (21, 22, 23) are provided. On each section (41), the DNA fragments are fixed at their one ends and comprises two portions (21a, 21b) each consisting of a common base sequence (common to corresponding portions of DNA fragments fixed onto the other sections) and a portion (21b, 22b, 22c), which has a base sequence specifically selected for each section. In the following, the former portion of a common base sequence is referred to as "common oligonucleotide portion", and the latter portion is referred to as "specific polynucleotide portion".

Each of the DNA fragments fixed onto the section preferably has two common oligonucleotide portions (21a, 21c) and one specific polynucleotide portion (21b) intervening the common oligonucleotide portions. The common oligonucleotide portions (21a, 21b) are preferably different from each other in their base sequences. There is no need of predetermining the base sequence of the common oligonucleotide portion. The common oligonucleotide portion preferably has base units of 10 to 100. One common oligonucleotide portion tray be composed of two or more sub-common oligonucleotide portions.

The DNA fragment to be fixed onto the substrate is preferably prepared by the steps of preparing a various recombinant DNA plasmids from extrinsic DNAs of mammals such as human being and mouse, yeast, lactic acid bacterium, *Escherichia coli*, and *Actinomyces*; converting the recombinant DNA plasmids into insert DNAs, and sub-cloning the insert DNAs. To each end of thus prepared DNA fragment (consisting of specific polynucleotide portion) is attached a different common oligonucleotide portion which is prepared from a vector (which is prepared by sub-cloning procedure). The DNA fragment can be prepared by other processes, such as synthetic process, provided that the DNA fragment comprises one or more common oligonucleotide portions and a specific polynucleotide portion.

The substrate of the DNA chip of the invention can be any of known substrates or their equivalent materials. The substrate preferably has a low hydrophilic surface or a hydrophobic surface. The substrate may have a plain surface or a surface having many fine concaves and convexes.

The substrate can be prepared from glass, ceramics, polymer materials (e.g., polyethylene terephthalate, cellulose acetate, polycarbonate of Bisphenol A, polystyrene, poly(methyl methacrylate), silicon, active carbon, and porous materials (e.g., porous glass, porous ceramics, porous silicon, porous active carbon, cloth, knitted cloth, non-woven cloth, filter paper, and membrane filter). Polymer materials, glass, and silicon are preferably employed.

Generally, the substrate is employed in the form of a sheet (or a film), and preferably has a thickness in the range of 100 to 2,000 µm. The porous substrate preferably has pores having a mean pore size in the range of 2 to 1,000 nm, more preferably, 2 to 500 nm.

The substrate is preferably pre-treated with poly-L-lysine, polyethyleneimine, or polyalkylamine. The treatment with poly-L-lysine is particularly preferred. The substrate is also preferably pre-treated with a coupling agent having an amino, aldehyde or epoxy group. The treatment of poly-L-lysine and a coupling agent in combination can be also utilized. The surface pre-treatment can increase electrostatic affinity of the DNA fragment to the surface of a hydrophobic or poor hydrophilic surface of the substrate.

The surface-treated substrate can be covered with a layer of an electro-charging hydrophilic polymer or a crosslinked polymer layer. The coverage with the polymer layer can make the pre-treated surface of the substrate physically smooth. Such polymer may be incorporated into the substrate.

In the preparation of the DNA chip of the invention, the aqueous DNA fragment solution is spotted onto each section of the substrate. The aqueous DNA fragment solution can contain a hydrophilic polymer material. Generally, the aqueous DNA fragment solution is once divided by placing the solution in a 96-well or 384-well plastic plate, and the divided small portion is then spotted onto each section (area) of the substrate by means of a spotter apparatus.

The fixation of DNA fragment can be stabilized by the use of a hydrophilic polymer. Preferably, one or both of the following treatments can be utilized to perform more stabilized fixation:
(1) Incorporation of an electro-charging group such as amino, aldehyde, thiol, or biotin into the DNA fragment at one end. The amino group is most preferred.
(2) Post-treatment of the spotted aqueous DNA fragment-polymer solution with heat treatment, irradiation with ultraviolet rays, or application of sodium borohydride or a Schiff base. The post-treatments can be utilized in combination. Preferred post-treatment is a combination of heat treatment and ultraviolet irradiation. It is considered that the post-treatment produces cross-linking between the electro-charging group of the DNA fragment and the pre-treated surface of the substrate, so that the fixation is more stabilized.

The hydrophilic polymer can be a cationic, anionic or amphoteric polymer. A nonionic polymer is also employable. The hydrophilic polymer preferably shows a high electrostatic affinity to the DNA fragment and does not disturb hybridization between the fixed DNA fragment and a target DNA fragment to be analyzed.

Examples of the hydrophilic polymers include poly-(1,4-diazoniabicyclo[2.2.2]octane-1,4-diylmethylene-1,4-phenylene methylene chloride), polyacrylamide, polyethylene glycol, carboxymethyl cellulose, sodium polyacrylate, sodium polyvinylbenzenesulfonate, and albumin.

Generally, the positive effect of the hydrophilic polymer to the fixation of DNA fragment to the substrate is the strongest when it is a cationic polymer. Then, the strength lowers in order from the cationic polymer, a nonionic polymer, an anionic polymer of COO⁻ type, an amphoteric polymer, and an anionic polymer of SO³⁻ type. The hydrophilic polymer preferably has a molecular weight in the range of 10³ to 10⁶. The DNA fragment-hydrophilic polymer solution preferably contains the hydrophilic polymer in the range of 0.1 to 2 vol.%, more preferably 0.5 to 1 vol.%.

The DNA fragment to be spotted preferably is in an amount of several ng or less for each section. Each section is preferably separated from the adjoining section at a space of 1.5 mm or less, preferably a space of 100 to 300 µm. One section preferably has a diameter of 50 to 300 µm. The aqueous solution to be spotted preferably in the range of 100 pL to 1 µL, more preferably 1 to 100 nL.

The spotted DNA fragment-hydrophilic polymer is incubated, if appropriate. After the incubation, unfixed free DNA fragments and poorly fixed DNA fragments are preferably washed out. Thus prepared DNA chip can be stored generally for several weeks to several months.

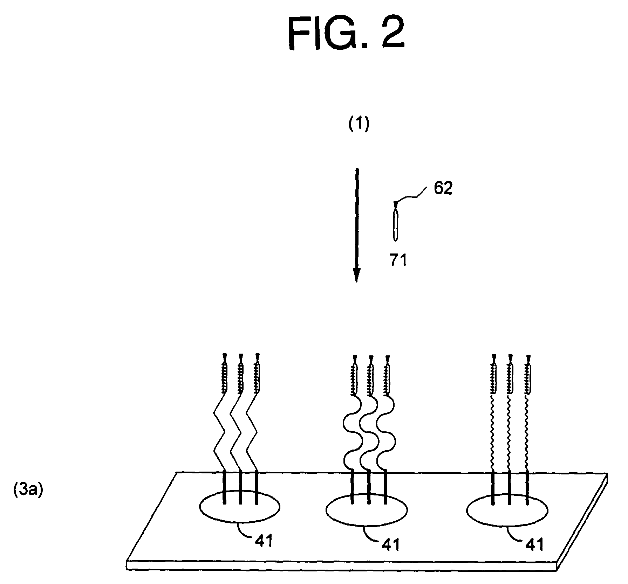

The DNA chip of the invention is employable for more accurately analyzing the nature of a target DNA fragment. For instance, the DNA chip of the invention is favorably employable in the mode illustrated in Fig. 2.

In Fig. 2, an aqueous solution containing an oligonucleotide (71) which is complementary to the common oligonucleotide portion (21a) and has a label (62) is uniformly brought into contact with each of the DNA fragment-fixed sections (41) in an amount of sufficiently performing hybridization with essentially whole DNA fragments fixed on each section. Subsequently, the strength of the label in each section is measured to determine an effective number of DNA fragments fixed in each section in terms of a relative detection sensitivity value. Thus, the desired relative detection sensitivity on each section can be determined. In the DNA chip illustrated in Fig. 2, the three sections have the same relative sensitivity, namely, 1:1:1. This method of determination of a relative detection sensitivity is favorably employable by manufacturers of DNA chip.

The oligonucleotide that is complementary to the common oligonucleotide portion of the fixed DNA fragment and has a label (referred hereinafter as to "labeled oligonucleotide") is preferably prepared by extracting mRNA from a cell or organ of eucaryote and incorporating a dNTP label into it by reverse transcription reaction to give a labeled cDNA fragment, or attaching a label to a synthesized oligonucleotide. The labeled oligonucleotide preferably has 10 to 100 base units.

The contact of the aqueous labelled oligonucleotide solution to the DNA fragment-fixed sections can be performed by dipping the DNA chip into the aqueous labeled oligonucleotide solution, or uniformly covering the DNA chip with the aqueous labeled oligonucleotide solution. The hybridization proceeds upon contact between the DNA fragment fixed on the DNA chip and the labeled oligonucleotide. The contact is preferably performed at a temperature between room temperature and 70°C, for a period of 6 to 20 hours. After the contact is complete, the DNA chip is preferably washed with a mixture of a surface active agent and a buffer solution to remove unreacted oligonucleotide. The surface active agent preferably is sodium dodecylsulfate (SDS). The buffer solution preferably is a citrate buffer solution, a phosphate buffer solution, a borate buffer solution, a Tris buffer solution, or a Good's buffer solution. Most preferred is a citrate buffer solution.

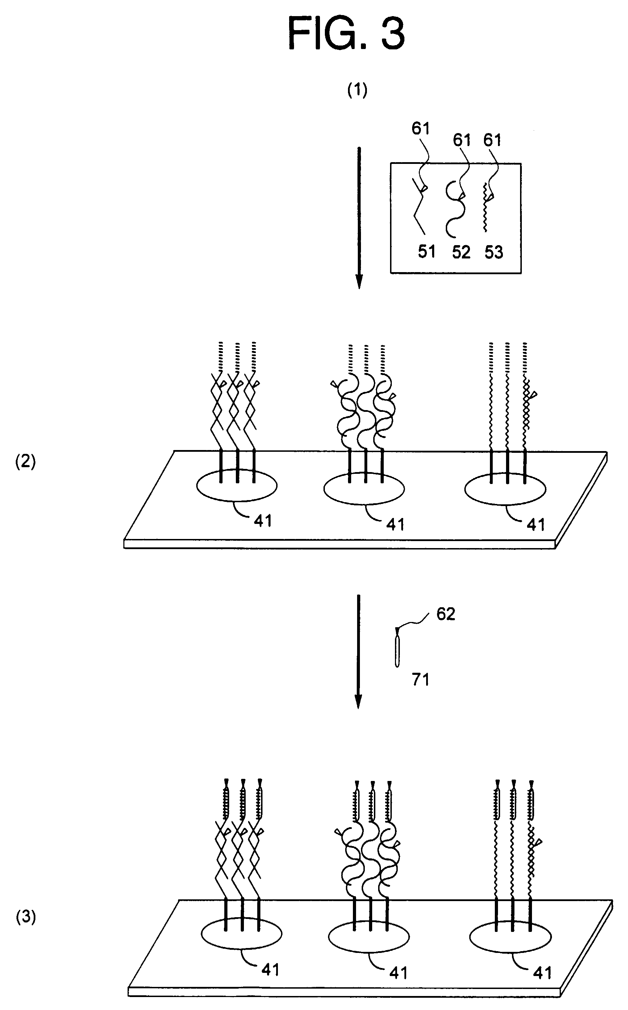

Fig. 3 illustrates another mode of the utilization of the DNA chip of the invention. This mode is favorably employed by the practitioner of DNA analysis.

In the first procedure (Procedure A), an aqueous sample solution containing predetermined amounts of three different DNA fragments (51, 52, 53) which are complementary to the specific polynucleotide portions of the DNA fragments fixed to the substrate, respectively, and have the same label (61) is brought into contact with the sections (41) of the DNA chip of the invention, so as to perform hybridization. Subsequently, the strength of label on each section is measured to determine a relative detection sensitivity of each section.

In the second procedure (Procedure B), the sections (41) of the DNA chip used in Procedure A are brought into contact with an aqueous solution containing a DNA, fragment (71) which is complementary to the common oligonucleotide portion and has a label (62) differing from the label (61) employed in Procedure A in an amount of sufficiently performing hybridization with essentially whole DNA fragments fixed on each section. Subsequently, the strength of label on each section is measured to determine a relative detection sensitivity of each section.

Procedure B can be performed in advance of Procedure A, if appropriate. Otherwise, Procedures A and B are performed almost in one procedure.

The labeled DNA fragment which is complementary to the specific polynucleotide portion of the fixed DNA fragment can be prepared by extracting mRNA from cells or organs of an eucaryote of the same genus as that of the eucaryote from which the specific polynucleotide portion is prepared, incorporating a dNTP label into it by reverse transcription reaction to give a labeled cDNA fragment. For instance, if the eucaryote from which the specific polynucleotide portion is obtained is yeast, a labeled CDNA fragment can be prepared by synthesis using as a mold mRNA extracted from yeast of wild type or TUPI defective yeast. If the extraction of mRNA is difficult, particularly, in the case that the origin is procaryote, a labeled mRNA or a DNA fragment produced by PCR utilizing a labeled primer and a labeled dNTP can be employed.

The label can be RI (i.e., radioisotope), fluorescence, biotin, or chemically fluorescent label. In the case of two fluorescence labels are employed in the case illustrated in Fig. 3, the positions of peak strengths of the fluorescence labels are preferably apart from each other by at least 10 nm.

Any fluorescence material can be employed, provided that it can be attached to a base unit of a DNA fragment. Preferred are a cyanine dye (e.g., Cy3 and Cy5 belonging to Cy Dye (trademark) series), a rhodamine 6G dye, N-acetoxy-N²-acetyaminofluorene (AAF), and AAIF (i.e., iodo derivative of AAF). The combination of flurorescences having a difference of position of peak strength by at least 10 nm preferably is a combination of Cy5 and rhodamine 6G dye, a combination of Cy3 and fluorescein, or a combination of rhodamine 6G and fluorescein.

In the determination of relative sensitivity of the DNA chip of the invention which has two or more common oligonucleotide portions, two or more hybridization procedures can be performed using two or more labeled oligonucleotides having different labels and different base sequences, so that the accuracy of the determination of relative sensitivity can be increased.

The kit of the invention comprises a DNA chip (see Fig. 1) of the invention and a labeled oligonucleotide (see 71 of Fig. 2). The labeled oligonucleotide can have any kinds of label, but an oligonucleotide labeled with fluorescence material is preferably employed.

The present invention is further described by the following examples.

### [Example 1] -- Preparation of DNA chip

### (1) Pretreatment of slide glass

A slide glass (25 mm x 75 mm) was dipped into an aqueous solution of 50 g of sodium hydroxide in a mixture of 150 mL of distilled water and 200 mL of ethanol for 1 hour. The slide glass was recovered from the solution, washed, and placed in an aqueous solution of poly-L-lysine (10 vol.% solution, available from Sigma Corp.) for 1 hour. The slide glass was then subjected to centrifugal treatment using a centrifugal apparatus for plates, and then dried at room temperature. Thus processed slide glass was employed in the below-mentioned procedures.

### (2) Preparation of DNA fragment to be fixed on the slide glass

Recombinant DNA plasmids prepared from various genes originating from yeasts were sub-cloned using M13 phage and subjected to PCR to give various PCR products. Each of the various PCR products (i.e., DNA fragments) has three base sequence potions, that is, a specific base sequence portion originating from the specific yeast, a common base sequence portion originating from M13 phage and attaching to one end of the specific base sequence portion, and another base sequence portion originating from M13 phage and attaching to another end of the specific base sequence portion.

### (3) Fixation of DNA fragment

Eight DNA fragments produced in (1) above were employed. One mL of an aqueous solution of one DNA fragment (0.5 mg/mL, in 3 x SSC, that is, a solution obtained by diluting one portion of the standard brine and citrate buffer solution with two portions of water) was diluted with 3 x SSC, to give a series of diluted solutions (namely, a two times diluted solution, a four times diluted solution, a eight times diluted solution, a sixteen times diluted solution, and a thirty-two times diluted solution).

To each of the obtained DNA fragment solutions was added CMC (carboxymethyl cellulose) to give a solution containing 1 vol.% CMC (concentration of the finally prepared solution). Thus obtained six DNA fragment solutions of the DNA fragment concentrations of 0.50 ng/nL, 0.25 ng/nL, 0.13 ng/nL, 0.063 ng/nL, 0.031 ng/nL, and 0.016 ng/nL. Each of the DNA fragment solutions was spotted on each of six sections (A_{₁}, A₂, A₃, A₄, A₅ and A₆) of the slide glass surface in an amount of 1 nL using a spotter apparatus.

Each of the other seven fragments was also processed in the same manner as above, to give six DNA fragment solutions all containing CMC. Each of the six DNA fragment solutions for the other seven fragments was then spotted on each of other six sections for each seven fragments, namely, B₁ to B₆, C₁ to C₆, D₁ to D₆, E₁ to E₆, F₁ to F₆, G₁ to G₆, and H₁ to H₆, in the manner illustrated in the following table 1.

**Table 1**

| | | | | | | | |
|---|---|---|---|---|---|---|---|
| A₁ | B₁ | C₁ | D₁ | E₁ | F₁ | G₁ | H₁ |
| A₂ | B₂ | C₂ | D₂ | E₂ | F₂ | G₂ | H₂ |
| A₃ | B₃ | C₃ | D₃ | E₃ | F₃ | G₃ | H₃ |
| A₄ | B₄ | C₄ | D₄ | E₄ | F₄ | G₄ | H₄ |
| A₅ | B₅ | C₅ | D₅ | E₅ | F₅ | G₅ | H₅ |
| A₆ | B₆ | C₆ | D₆ | E₆ | F₆ | G₆ | H₆ |

The slide glass having various spotted DNA fragment solutions was heated to 70°C for one hour and was irradiated with ultraviolet rays at an irradiation strength of 120 KJ. The irradiated slide glass was then placed in an aqueous 0.1 M sodium borate solution containing 70 mM succinic anhydride for 10 min., shaken intermittently, placed in boiling MilliQ water, placed in ethanol, and dried at room temperature.

### [Example 2] -- Determination of relative sensitivity of DNA chip

### (1) Preparation of labeled oligonucleotide

An oligonucleotide of 24 mers was labeled with a fluorescence label agent (FluoroLink Cy3-dCTP, available from Amasham Pharmacia Biotech, PA 53022) at its 5' terminal.

### (2) Hybridization and measurement of fluorescence strength

The labeled oligonucleotide obtained in (1) above was dispersed in a hybridization solution (mixture of 4 x SSC and 10 wt.% SDS (sodium dodecyl sulfate) solution) to give a dispersion containing 2 pico mol./µL of the oligonucleotide. Thus obtained dispersion was slowly placed on the DNA chip prepared in Example 1. The DNA chip was incubated in a moisture chamber at 60°C for 20 hours, for performing hybridization. The incubated DNA chip was then immersed in a mixture of 0.1 wt.% SDS solution and 2xSSC solution, and successively washed with 0.1 wt.% SDS solution and 2xSSC solution, 0.1 wt.% SDS solution and 0.2xSSC solution and 0.2xSSC solution. Thus washed DNA chip was subjected to centrifugal processing at 600 rpm for 20 seconds, and dried at room temperature.

The DNA chip after hybridization was subjected to fluorometric measurement using a fluorescence scanning apparatus, to measure a fluorescence strength in each section. Then, the desired relative fluorescence strength was calculated. The calculated relative fluorescence strengths are set forth in Table 2.

**Table 2**

| Section | A₁ | B₁ | C₁ | D₁ | E₁ | F₁ | G₁ | H₁ |
|---|---|---|---|---|---|---|---|---|
| Relative Strength | 1.24 | 1.26 | 1.28 | 1.00 | 1.26 | 1.33 | 1.02 | 1.15 |

The results set forth in Table 2 means that the sensitivity values of each of A₁ to H₁ sections differ from each other, while the sensitivity values are expected to be the same. The results are understood to indicate that the relative sensitivity can be determined by the above-described procedures.

In the following Table 3, the relative sensitivity values on the sections A₁ to A₆ are given. A satisfactory linearity of the relative sensitivity values against the amount of the spotted DNA fragment is seen.

**Table 3**

| Section | A₁ | A₂ | A₃ | A₄ | A₅ | A₆ |
|---|---|---|---|---|---|---|
| Relative sensitivity | 3.01 | 2.59 | 2.01 | 1.53 | 1.37 | 1.00 |

## Claims

1. A DNA chip comprising a substrate which has plural sections on its surface and a group of DNA fragments fixed at their one ends onto each of the sections, a group of DNA fragments fixed onto one section having essentially the same base sequence, wherein a DNA fragment comprises at least two portions, one portion having a base sequence specifically selected for each section and the other portion having a common base sequence.

2. The DNA chip of claim 1, wherein the DNA fragment comprises three portions, one portion having a base sequence specifically selected for each section, one of the other two portions having a common base sequence and being placed between the substrate and one end of the portion of specifically selected base sequence, and another of the other two porions having a common base sequence and being attached to the other end of the portion of specifically selected base sequence.

3. A kit which has a DNA chip comprising a substrate which has plural sections on its surface and a group of DNA fragments fixed at their one ends onto each of the sections, a group of DNA fragments fixed onto one section having essentially the same base sequence, wherein a DNA fragment comprises at least two portions, one portion having a base sequence specifically selected for each section and the other portion having a common base sequence, and a labelled oligonucleotide having a portion of a base sequence which is complementary to the common base sequence of the portion of the DNA fragment fixed onto the substrate.

4. The kit of claim 3, wherein the labelled oligonucleotide comprises an oligonucleotide and a fluorescence label.

5. A method of determining a relative sensitivity on each section, which comprises the steps of:
bringing a DNA chip comprising a substrate which has plural sections on its surface and a group of DNA fragments fixed at their one ends onto each of the sections, a group of DNA fragments fixed onto one section having essentially the same base sequence, wherein a DNA fragment comprises at least two portions, one portion having a base sequence specifically selected for each section and the other portion having a common base sequence, into contact with a labelled oligonucleotide having a portion of a base sequence which is complementary to the common base sequence of the portion of the DNA fragment fixed onto the substrate, in the presence of an aqueous medium, so as to combine the labelled oligonucleotide to each of the DNA fragments through hybridization; and
detecting the labelled oligonucleotide combined to the DNA fragment on each section of the DNA chip.

6. The method of claim 5, wherein the labelled oligonucleotide comprises an oligonucleotide and a fluorescence label, and the detection is performed by fluorometry.
